# EUROPEAN PATENT SPECIFICATION

(11) **EP 0 700 429 B1**
(45) Date of publication and mention of the grant of the patent: **11.10.2000**
(21) Application number: 93911075.5
(22) Date of filing: 30.04.1993
(51) Int. Cl.: C12N 5/00, C12N 11/00, A01N 1/02, A61M 35/00, A61F 2/14, A61L 27/00

(54) **RETINAL PIGMENT EPITHELIUM TRANSPLANTATION**
TRANSPLANTATION DES RETINALEN PIGMENTEPITHELS
TRANSPLANTATION D'EPITHELIUM PIGMENTAIRE DE LA RETINE

(43) Date of publication of application: 13.03.1996
(73) Proprietor: PHOTOGENESIS Incorporated, Los Angeles, CA 90069 (US)
(72) Inventor: Liu, Yao, Haddonsville, NJ, 08033 (US); Hughes, Stephen E., Delmar, New York, 12054 (US); Silverman, Martin S., St Louis, Missouri, 63108 (US)
(74) Representative: Littolff, Denis
(86) International application number: US9304245
(87) International publication number: WO9425569

(56) References cited:
- WO-A-91/02499
- US-A- 4 495 288
- US-A- 4 927 676
- US-A- 4 963 489
- US-A- 5 000 963
- Polyscience, issued January 1990, "Biodegradable Polymers", page 365, see entire document.
- Graefe's Archives for Clinical and Experimental Ophthalmology, Volume 224, issued 1986, RADTKE et al., "Pharmacological Therapy for Proliferative Vitreoretinopathy", pages 122-125, see entire document.
- Investigative Ophthalmology & Visual Science, Volume 30, No. 8, issued August 1989, SILVERMAN et al., "Transplantation of Photoreceptors to Light-Damaged Retina", pages 1684-1690, see entire document.
- Current Eye Research, Volume 9, No. 2, issued 1990, SILVERMAN et al., "Photoreceptor Rescue in the RCS Rat without Pigment Epithelium Transplantation", pages 183-191, see entire document.
- American Journal of Ophthalmology, Volume 80, No. 3, Part II, issued September 1975, MUELLER-JENSEN et al., "Autotransplantation of Retinal Pigment Epithelium in Intravitreal Diffusion Chamber", pages 530-537, see entire document.
- Exp. Eye Res., Volume 47, issued 1988, LI et al., "Transplantation of Retinal Pigment Epithelial Cells to Immature and Adult Rat Hosts; Short- and Long-Term Survival Characteristics", pages 771-785, see entire document.
- Investigative Ophthalmology & Visual Science, Volume 30, No. 3, issued March 1989, LOPEZ et al., "Transplanted Retinal Pigment Epithelium Modifies the Retinal Degeneration in the RCS Rat", pages 586-589, see entire document.
- Exp. Eye Res., Volume 48, issued 1989, SHEEDLO et al., "Functional and Structural Characteristics of Photoreceptor Cells Rescued in RPE-Cell Grafted Retinas of RCS Dystrophic Rats", pages 841-854, see entire document.
- Investigative Ophthalmology & Visual Science, Volume 24, issued July 1983, ANDERSON et al., "Retinal Detachment in the Cat: the Pigment Epithelial-Photoreceptor Interface", pages 906-926, see entire document.
- Investigative Ophthalmology & Visual Science, Volume 26, issued November 1985, MAYERSON et al., "An Improved Method for Isolation and Culture of Rat Pigment Epithelial Cells", pages 1599-1609, see entire document.

## Description

### BACKGROUND OF THE INVENTION

The present invention relates in general to cell and tissue transplantation techniques. More particularly, the present invention is directed to techniques for transplanting populations of retinal pigment epithelium (RPE) cells as a monolayer to the subretinal region of the eye, and to methods for preparing implants comprising monolayers of RPE cells for transplantation.

The retina is the sensory epithelial surface that lines the posterior aspect of the eye, receives the image formed by the lens, transduces this image into neural impulses and conveys this information to the brain by the optic nerve. The retina comprises a number of layers, namely, the ganglion cell layer, inner plexiform layer, inner nuclear layer, outer plexiform layer, outer nuclear layer, photoreceptor inner segments and outer segments. The outer nuclear layer comprises the cell bodies of the photoreceptor cells with the inner and outer segments being extensions of the cell bodies. The choroid is a vascular membrane containing large branched pigment cells that lies between the retina and the sclerotic coat of the vertebrate eye. Atop the choroid is a membrane 1-5 microns in thickness essentially composed of collagen, known as Bruch's membrane.

Immediately between Bruch's membrane and the retina is the retinal pigment epithelium which forms an intimate structural and functional relationship with the photoreceptor cells. Among the functions performed by RPE cells is the phagocytosis of outer segment debris produced by the photoreceptors. It is believed that failure of the RPE cells to properly perform their functions such as digestion of outer segment debris leads to the eventual degeneration and loss of photoreceptor cells.

In the leading causes of visual impairment in western industrialized countries, such as age-related macular degeneration (AMD), both photoreceptors and the underlying RPE are compromised, or have degenerated. A further aspect of AMD is the frequent appearance of subretinal neovascular membranes which grow through the Bruch's membrane and the RPE and tend to hemorrhage and leak fluids into the subretinal space.

In an effort to recover what was previously thought to be an irreparably injured retina, researchers have suggested various forms of grafts and transplantation techniques, none of which constitute an effective manner for reconstructing a dystrophic retina. The transplantation of retinal cells to the eye can be traced to a report by Royo et al., Growth 23: 313-336 (1959) in which embryonic retina was transplanted to the anterior chamber of the maternal eye. A variety of cells were reported to survive, including photoreceptors. Subsequently del Cerro was able to repeat and extend these experiments (del Cerro et al., Invest. Ophthalmol. Vis. Sci. 26: 1182-1185, 1985). Soon afterward Turner, et al. Dev. Brain Res. 26:91-104 (1986) showed that neonatal retinal tissue could be transplanted into retinal wounds.

Li and Turner, Exp. Eye Res. 47:911 (1988) have proposed the transplantation of retinal pigment epithelium (RPE) into the subretinal space as a therapeutic approach in the RCS dystrophic rat to replace defective mutant RPE cells with their healthy wild-type counterparts. According to their approach, RPE were isolated from 6- to 8-day old black eyed rats and grafted into the subretinal space by using a lesion paradigm which penetrates through the sclera and choroid. A 1 ml bolus injection of RPE (40,000 - 60,000 cells) was made at the incision site into the subretinal space by means of a 10 ml syringe to which was attached a 30 gauge needle. However, while this technique is marginally appropriate for immature RPE cells, with mature cells it leads to activation and transformation of these cells which damages eye and retinal tissue.

Lopez et al., Invest. Ophthalmol. Vis. Sci. 30: 586-589, 1989, also reported a procedure for the transplantation of dissociated RPE cells. In this procedure, RPE cells were obtained from normal, congenic, pigmented rat eyes by trypsin digestion. These freshly harvested, dissociated RPE cells were injected into the subretinal area of the eyes of dystrophic RCS rats via an incision through the sclera, choroid and neural retina. Comparable to the Li and Turner approach discussed above, this procedure destroys the organized native structure of the transplanted RPE cells, which take the form of a confluent monolayer in a healthy eye. Moreover, the procedure is of questionable value for the transplantation of mature RPE cells. When mature RPE cells are transplanted in dissociated form, experimental results indicate that they are likely to become activated, migrate into the subretinal space, and as noted by Lane, C., et al., Eye (1989) 3, 27-32, invade the retina and vitreous. This activation of the transplanted, mature RPE cells can result in such pathologies as retinal pucker, massive subretinal fibrosis, retinal rosette formation, retinal detachment, and proliferative vitreoretinopathy.

The difficulties discussed above associated with the transplantation of mature RPE cells is problematic for del Cerro et al., Neurosci. Lett. 92 : 21-26, 1988, also reported a procedure for the transplantation of dissociated neuroretinal cells. In this procedure, the donor retina is cut into small pieces, incubated in trypsin for 15 minutes, and triturated into a single cell suspension by aspirating it through a fine pulled pipette. Comparable to the Li and Turner approach discussed above, this procedure destroys the organized native structure of the transplant, including the donor outer nuclear layer ; the strict organization of the photoreceptors with the outer segments directed toward the pigment epithelium and the synaptic terminals facing the outer plexiform layer are lost. Furthermore, no means of isolating and purifying any given population of retinal cells (e.g. photoreceptors) from other retinal cells was demonstrated.

It is believed by the present inventor that it is necessary to maintain the photoreceptors in an organized outer nuclear layer structure in order to restore a reasonable degree of vision. This conclusion is based on the well known optical characteristics of photoreceptor (outer segments act as light guides) and clinical evidence showing that folds or similar, even minor disruptions in the retinal geometry can severely degrade visual acuity.

In a prior European Patent 0 486 589, the Applicant has described a first approach for a new method, in which essential criterion was that the retinal pigment epithelium cells (RPE) were to be laminated to a support whereby the polarity and the organization of said cells were maintained in the graft in order to restore a reasonable degree of vision, such a lamination of RPE cells in monolayer form being not suggested in any of the prior documents such as LOPEZ, as cited above, which reports a procedure for the transplantation of dissociated RPE cells.

Now the present invention has for its object a significant improvement in this technique, in which the method for the preparation of a population of retinal epithelium cells for transplantation to the subretinal area of an eye of a host comprises :
a) providing donor tissue comprising retinal pigment epithelium cells,
b) harvesting retinal pigment epithelium cells from said donor tissue,
c) culturing said harvested retinal pigment epithelium cells on a substrate suitable for attachment and growth of a monolayer of retinal pigment epithelium cells, and
d) removing the monolayer of cultured retinal pigment epithelium cells from said substrate.

The differences and advantages of cultured (i.e. separately grown) cells of the invention can be emphasized as follows :

Whereas it is well known that RPE cells in the eye form a monolayer, what is now claimed is a monolayer of cultured RPE cells on a thin collagen substrate, in which said substrate is thin enough not to interfere with normal eye tissue function, and in which said monolayer is formed from dissociated RPE cells which are cultured to a desired size and shape.

Now it should be taken into account the fact that the physical isolation of a natural RPE cells monolayer from a retina is difficult, and that the isolated pieces of RPE tissue are then small and irregular : this is the prior state of the art.

Reversely, cultured RPE grafts can be formed from a few cells of a person who is to receive the graft (autologous grafting), or the cultured RPE cells graft are sufficiently large that immunological matching can be done of allografts (from one human to another) to minimise the risk of an immune response. The cultured RPE cells grafts can be as large as desired and formed to a desired shape. The cells can be stored for long periods of time and can be genetically altered and potentially used for gene therapy.

Because RPE cells can be cultured and stored for long periods of time, a potentially unlimited and readily available supply of RPE cells grafts is now possible.

From this last view point it should reminded that, according to the prior art, naturally occurring RPE cells grafts are of limited supply since they must be harvested from cadavers (dead people), and further, RPE cells from a cadaver increases the chance of an immune response upon translation.

According to a preferred embodiment of this method, the harvested retinal pigment epithelium cells are apposed as a monolayer to a non toxic flexible support that, upon transplantation to the subretinal area and exposure to conditions which result in a breakdown of at least a portion of the non toxic flexible support, will not impede normal eye tissue function of the eye of the host and the transplanted population.

Preferably, the donor tissue comprises mature retinal pigment epithelium cells, and is analogous to the host.

Preferably also, the support comprises a layer of collagen less than about 100 microns in thickness.

According to a further feature of the invention, the retinal pigment epithelium cells have apical and basal surfaces and the support comprises a substrate of collagen and an overlayer of gelatin, and the basal surfaces of the harvested retinal pigment epithelium cells are apposed to the collagen and the apical surfaces of the retinal pigment epithelium cells are apposed to the gelatin. Generally speaking, the support comprises a material degradable by exposure to heat or body fluids, and possibly also one or more of a trophic factor, an immunodepressant, an anti-inflammation agent, an anti-angiogenic factor, an anti-glial agent or an anti-mitotic factor.

This invention has also for object an implant for the transplantation to the subretinal area of an eye of a host of a monolayer of retinal pigment epithelium cells, in which the characteristic feature of the implant is to comprise cultured retinal pigment epithelium cells.

The invention comprises also such an implant, in which said cultured retinal pigment epithelium cells are laminated on a thin collagen substrate.

According to a preferred modification of such an implant, it comprises a laminate of a monolayer of retinal pigment epithelium cells and a non toxic flexible support which, upon transplantation to the subretinal area and exposure to conditions which result in a breakdown of at least a portion of the non toxic support, will not impede normal eye tissue function.

Preferably, in said implant, the retinal pigment epithelium cells comprise mature retinal pigment epithelium cells, which are analogous to the host : preferably also, in said implant, the support comprises a material degradable by exposure to heat or body fluids, and comprises a layer of collagen less than 100 microns of thickness.

Finally, according to a further feature of the invention, in said implant, the monolayer of retinal pigment epithelium cells have apical and basal surfaces, and the support comprises a substrate of collagen and an overlayer of gelatin, the basal surfaces of the monolayer of retinal pigment epithelium cells being laminated to the collagen and the apical surfaces of the retinal pigment epithelium cells being laminated to the gelatin.

### SUMMARY OF THE INVENTION

Among the objects of the present invention, therefore, may be noted the provision of a method for preparation of a graft for use in the reconstruction of a dystrophic retina ; the provision of such a method which conserves relatively large expanses of the tissue harvested from a donor eye ; the provision of such a method in which the polarity and organization of the cells at the time of harvest are maintained in the graft ; the provision of a graft for use in the reconstruction of a dystrophic retina ; membranes. In practice, removal of the neovascular membrane results in removal of the native RPE and Bruch's membrane as well.

A critical impediment to the transplantation of RPE cells as a monolayer is the fragility of the intercellular structure of RPE relative to the rigors of manipulation during transplantation to the subretinal area. Moreover, providing satisfactory support to the RPE cells during this process is complicated by the fact that the support must either be removed subsequent to transplantation, to avoid compromising metabolic exchange between the choroid and the overlying retina, or be compatible with such ongoing physiological activity. Thus, a method is needed wherein an implant comprising a monolayer of RPE cells is prepared and transplanted in which the component supporting the monolayer of RPE cells, upon transplantation to the subretinal area and exposure to a set of predetermined conditions, does not impede normal eye tissue function. Further, a Bruch's-like membrane for attachment of the transplanted RPE cells will be needed in those cases where neovascularization has occurred and the native Bruch's membrane is removed.

### SUMMARY OF THE INVENTION

Among the objects of the present invention, therefore, may be noted the provision of a method for preparation of a population of RPE cells as a monolayer for use in the reconstruction of a dystrophic RPE; the provision of such a method which allows for use of mature, and in particular, autologous, mature RPE cells as donor tissue in the reconstruction of a dystrophic RPE; the provision of an implant for use in the reconstruction of a dystrophic retina; the provision of such an implant which does not interfere with normal eye tissue function after transplantation and maintains photoreceptors and their inner and outer segments by allowing for maintenance of the native organization of the photoreceptor; the provision of such an implant which provides a membrane for attachment of the population of RPE cells especially when the native Bruck's membrane has been removed; and the provision of a method for transplantation of such implants to the subretinal area of an eye.

Briefly, therefore, the present invention is directed to a method for the preparation of a population of RPE cells for transplantation to the subretinal area of a host eye. The method comprises providing donor tissue comprising RPE cells, harvesting from that tissue RPE cells, and apposing the harvested RPE cells as a monolayer to a non-toxic, flexible support that, upon transplantation to the subretinal area and exposure to a set of predetermined conditions, will not impede normal eye tissue function of the host eye and the transplanted population.

The present invention is further directed to an implant for transplantation to the subretinal area of a host eye. The implant comprises a laminate of a monolayer of retinal pigment epithelium cells and a non-toxic, flexible support that, upon transplantation to the subretinal area and exposure to a set of predetermined conditions, will not impede normal eye tissue function.

The present invention is also directed to a method for transplanting to the subretinal area of a host's eye an implant comprising a monolayer of RPE cells. The method comprises providing an implant comprising a laminate of a monolayer of retinal pigment epithelium cells and a non-toxic, flexible support that, upon transplantation to the subretinal area and exposure to a set of predetermined conditions, will not impede normal eye tissue function, making an incision through the hosts eye, at least partially detaching the retina to permit access to the subretinal area, and positioning the implant in the accessed subretinal area.

### BRIEF DESCRIPTION OF THE DRAWINGS

Fig. 1 is a photograph (200X) of a cryostat section of a normal rat retinal and sub-retinal area;
Fig. 2(a, b, c and d) is a schematic showing the preparation and transplantation of a preferred embodiment of an implant comprising a monolayer of RPE cells mounted to a support;
Fig. 3 is a horizontal section through an eye illustrating a transchoroidal and scleral surgical approach;
Fig. 4 is a horizontal section through an eye illustrating a transcorneal surgical approach;
Fig. 5 is a photograph showing a view of the transplanted implant of an intact monolayer of mature human RPE cells positioned under the retina of a rodent whose cornea, pupil and lens have been removed, 2 weeks post-transplantation, as set forth in Example 1;
Fig. 6 is a photograph (40X) of a section of a rat retina and sub-retinal area showing an implant of an intact monolayer of a population of mature human REP cells 14 days after transplantation, as set forth in Example 1;
Fig. 7 is a higher magnification photograph (400X) of a section of a rat retina and sub-retinal area showing an implant of an intact monolayer of a population of mature human RPE cells 14 days after.transplantation, as set forth in Example 1;
Fig. 8 is a photograph (100X) of a section of a rat retina and sub-retinal area 14 days after transplantation of dissociated mature human RPE cells, as set forth in Example 2; and
Fig. 10 is a higher magnification photograph (300X) of a section of a rat retina and sub-retinal area 14 days after transplantation of dissociated mature human RPE cells, as set forth in Example 2.

### DETAILED DESCRIPTION

As used herein, the term "donor" shall mean the same or different organism relative to the host and the term "donor tissue" shall mean tissue harvested from or cultured from tissue harvested from the same or different organism relative to the host. Autologous tissue shall mean tissue harvested from or cultured from tissue harvested from the host organism. Mature RPE cells shall mean differentiated RPE cells derived from an organism that is not a fetus.

It is believed that in age-related macular degeneration (AMD), compromise or degeneration of the RPE cells which underlie and form a close structural and metabolic support for the photoreceptors, leads to the loss or destruction of viable photoreceptors. It has been discovered, however, that transplantation of a population of RPE cells as a monolayer, i.e., in essentially a two-dimensional array of cell bodies capable of engaging in cell-to-cell contact inhibition, allows the RPE cells to maintain largely normal characteristics of native, healthy retinal pigment epithelia, capable of providing structural and functional support to photoreceptor cells. Moreover, as illustrated in Example 2 below, it is believed that it is essential to transplant mature RPE cells as a monolayer in order to prevent activation of such cells. Activation leads RPE cells to transdifferentiate into wandering macrophages, fibroblast-like cells and other cell types, which cause a number of dystrophic effects in the eye and retina.

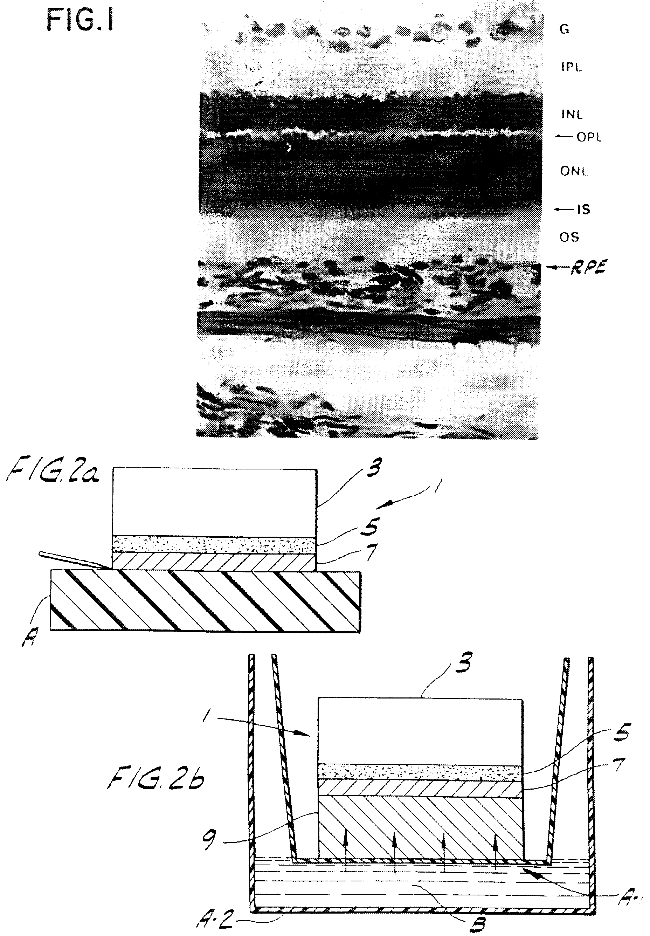

Fig. 1 is a photograph of a cryostat section of a normal rat retina and subretinal area. In Fig. 1 as well as subsequent figures, the retina or layers thereof, e.g., the ganglion cell layer ("G"), inner plexiform layer ("IPL"), inner nuclear layer ("INL"), outer plexiform layer ("OPL"), outer nuclear layer ("ONL"), inner segments ("IS"), outer segments ("OS"), and retinal pigment epithelium ("RPE"), are shown, respectively, from top to bottom.

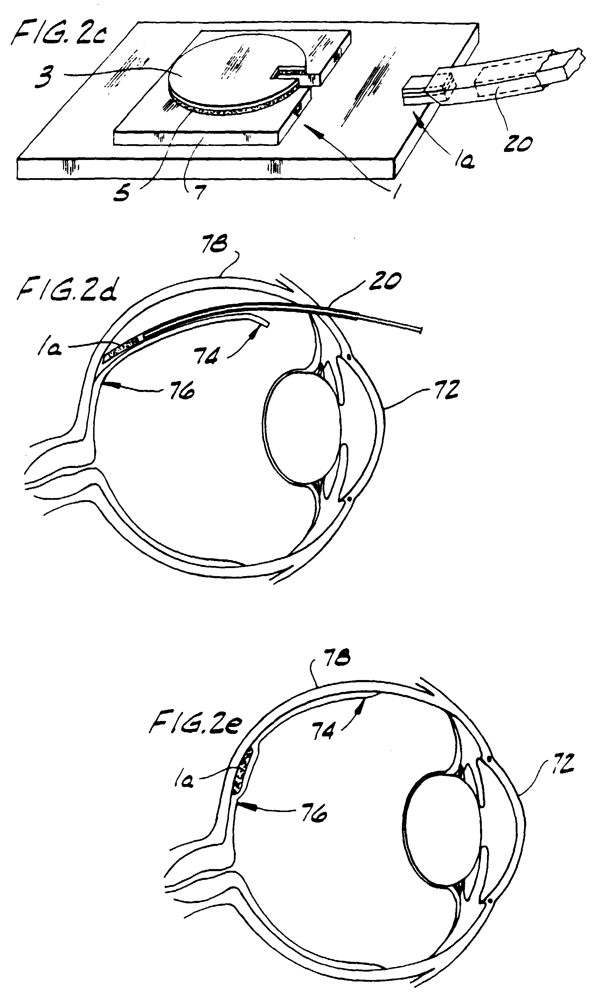

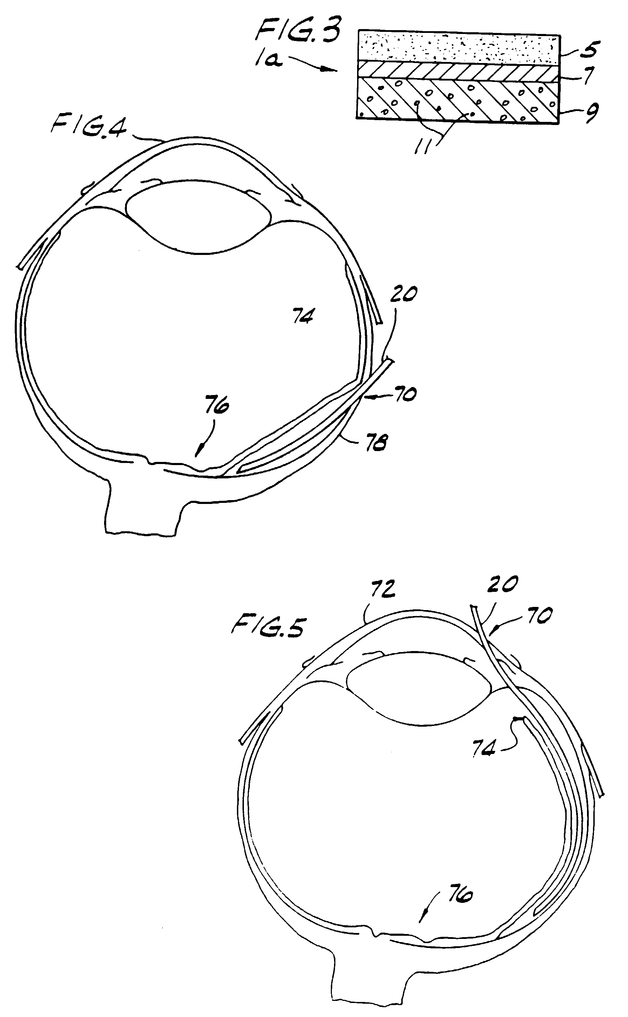

Referring now to Figs. 2 and 3, implants comprising a monolayer of RPE cells are prepared, in general, by harvesting RPE cells from donor tissue and apposing the harvested RPE cells as an intact monolayer to a non-toxic, flexible composition, or by seeding such a composition with a monolayer of dissociated RPE cells and allowing them to grow into a confluent layer. The flexible composition serves as a stabilizing support for the RPE cells during transplantation.

The implant constitutes a laminate of a monolayer of RPE cells and a support composition approximately 100 to 500 microns thick, and preferably 150-250 microns thick. The surface of the implant has a surface area greater than about 1 square millimeter, preferably greater than 2 square millimeters, and most preferably greater than 4 square millimeters, or as large as may be practically handled. Thus constructed, the implant may subtend a considerable extent of the sub-retinal surface.

In selecting donor tissue for the harvesting of RPE cells, it is noted that previously, transplantation of RPE cells was effectively limited to immature cells. Mature RPE cells transplanted according to prior art methods have been shown to undergo activation resulting in retinal pucker, sub-retinal fibrosis and proliferative vitreoretinopathy. However, by utilizing the procedures for implanting a monolayer of RPE cells according to this invention, transplanted mature RPE cells have successfully maintained essentially normal structure and function. Thus, the subject invention makes mature RPE cells available for transplantation, significantly increasing the supply of usable donor tissue beyond the narrowly limited supply of immature human donor tissue.

It is also noted that the RPE forms part of the blood-retinal barrier and is thus exposed to lymphocytic attack. Accordingly, use of autologous RPE cells, now possible even for mature RPE cells, is preferable, since it will avoid immunological complications in clinical applications. If non-autologous tissue is utilized, tissue typing and/or use of an immunosuppression regimen will generally be necessary to avoid rejection upon transplantation.

Donor tissue may be provided, for non-autologous, human RPE tissue, from eye banks, which make the RPE tissue available in connection with conducting corneal transplants. Harvesting donated tissue comprising non-autologous RPE cells can be accomplished by any suitable method. In general, harvesting of RPE cells may be carried out as set forth in Pfeffer, B.A., Chapter 10, "Improved Methodology for Cell Culture of Human and Monkey Retinal Pigment Epithelium", Progress in Retinal Research, Vol. 10 (1991); Ed. by Osborn, N. and Chader, J., or Mayerson, P.L., et al., "An Improved Method for Isolation and Culture of Rat Retinal Pigment Epithelial Cells", Investigative Ophthalmology & Visual Science, Nov., 1985, 26: 1599-1609, which are incorporated herein by reference. Specifically, to harvest non-autologous RPE cells, a donor eye is pinned by the optic nerve stump into an eye cage and placed in an eye jar immediately after corneal removal. The jar is then flooded to capacity with cold Delbecco's modified essential medium (DMEM) supplemented with 5% fetal bovine serum. After loose connective tissue and muscle have been carefully trimmed from the eye, it is placed upright on a sterile plate and the anterior segment with the adherent vitreous is lifted out of the eye cup. The neural retina is separated at the optic disc and removed. The shell is then washed with Hanks' balanced salt solution, Ca++ and Mg++ free, and treated with 0.25% trypsin for 30 min. at 37° C. The trypsin solution is aspirated from the shell and DMEM (GIBCO) is added. The RPE cells are released from Bruch's membrane by gently pipetting the culture medium in the shell.

For implants containing autologous tissue, RPE cells may be harvested by performing a biopsy following the procedures disclosed by Lane, C., et al. in Eye (1989) 3, 27-32.

The implant also comprises a support for the RPE cells so that the monolayer of RPE cells is less likely to be damaged and is more easily manipulated during the transplantation process. The support consists of a substrate, an overlayer or both, comprising a sheet or sheets of a non-toxic, flexible composition selected to provide mechanical strength and stability to the easily damaged monolayer of RPE cells. Because the support composition will be inserted into the eye as a laminate with the monolayer of RPE cells, the support is also comprised so that, upon transplantation to the subretinal area and exposure to a set of predetermined conditions, described herein, the support composition will not impede normal eye tissue function of the host eye or the population of transplanted RPE cells. If the harvested RPE cells are to be cultured before transplantation, the composition providing support to the monolayer of RPE cells during transplantation may optionally be capable of serving as the attachment substrate for the RPE cells during culturing.

A variety of compositions may be used as the support for the population of RPE cells, depending upon the specific set of conditions to which the composition will be exposed after transplantation. In short, the composition is selected either because any portion remaining within the subretinal area for more than approximately one week after transplantation is compatible with normal eye tissue function upon exposure to bodily fluids within the sub-retinal area, or for its susceptibility to elimination upon exposure to prescribed conditions.

An additional factor to consider in determining the make-up of compositions for support of the monolayer of RPE cells is whether Bruch's membrane has been or will be removed from the host eye prior to transplantation of the monolayer of RPE cells. Bruch's membrane serves to anchor the RPE cells in a healthy eye. Removal of Bruch's membrane may occur in cases of AMD where a subretinal neovascular membrane has formed and Bruch's membrane is removed as a consequence of the removal of the neovascular membrane.

In cases where Bruch's membrane is removed, the support composition will comprise a layer of collagen less than 100 microns in thickness, and preferably between 1 and 10 microns in thickness. The basal surfaces of the RPE cells attach readily to the collagen layer, which serves to anchor the RPE cells to the choroid in place of the removed Bruch's membrane. The layer of collagen also serves to inhibit the occurrence or reoccurrence of subretinal neovascularization through and around the transplanted RPE. Such a collagen layer is retained indefinitely in the sub-retinal area. However, it is known that Bruch's membrane is essentially comprised of collagen, and such microthin layers of collagen are permeable enough to avoid impeding normal eye tissue functions such as the metabolic exchange between the choroid and the retina. However, collagen compositions of such a minimal thickness are not strong enough to prevent buckling or distortion of the monolayer of RPE cells during transplantation. Thus, such microthin collagen materials need to be used in combination with additional supporting materials which will be eliminated after transplantation, if they are to form part of the support for the transplanted RPE cells.

If transplantation is carried out with the native Bruch's membrane intact, the host's RPE cells covering the area to receive the transplant of RPE cells may be physically and/or chemically debrided from the Bruch's membrane, for example, by applying collagenase to the native Bruch's membrane to loosen the host's RPE cells in order to permit their removal. The transplanted RPE cells may then anchor themselves directly to the Bruch's membrane.

Either to supplement a layer of collagen, as discussed above, or in cases where inclusion of a collagen anchor is not required, a support composition may be selected which is dissipated, for example, by exposure to a sufficient amount of heat or bodily fluids. Gelatin is an example of a preferred support material which is flexible, lacks toxicity to neural tissue and has the ability to dissolve at body temperature. urokinase is an enzyme specific to fibrin. Another alternative is to use biodegradable polymers such as ethylene-vinyl acetate copolymer (Elvax 40W, DuPont Chemical Co., Delaware); poly(glycolic) acid, poly(L-lactide-co-glycolide)(70:30 ratio) or poly(L- or DL-lactic) acids (low mol. wt.) (Polysciences, Inc., Warrington, PA., Data Sheet #365, 1990), which are flexible, non-toxic materials that slowly dissipate upon implantation and exposure to bodily fluids. See, e.g., Powell, E.M., Brain Research, 515 (1990) 309-311 and references cited in Polysciences, Inc. Data Sheet #365.

Advantageously, the gelatin or other support composition may additionally serve as a carrier for any of a number of trophic factors such as pharmacologic agents including immunosuppressants such as cyclosporin A, anti-inflammation agents such as dexamethasone, anti-angiogenic factors, anti-glial agents and anti-mitotic factors. Upon dissolution of the support composition, the factor or agent becomes available to impart the desired effect upon the surrounding tissue. The dosage can be determined by established experimental techniques.

With appropriate enzymatic digestion, using techniques disclosed by Pfeffer, B.A., Chapter 10, "Improved Methodology for Cell Culture of Human and Monkey Retinal Pigment Epithelium", Progress in Retinal Research, Vol. 10 (1991), at p.264, RPE cells are removed from the Bruch's membrane as intact sheets rather than as dissociated cells. Such a freshly harvested intact monolayer of RPE cells may be immediately apposed as an intact monolayer to a support, such as a thin collagen sheet, supplemented with an overlayer or substrate of gelatin, allowing several hours for adhesion prior to transplantation. This procedure may be useful for transplantation of RPE cells obtained in a biopsy, provided sufficient RPE tissue is obtained from the biopsy as an intact monolayer.

In most cases, however, it is preferable to culture the harvested RPE cells on an attachment substrate so that a monolayer of RPE cells may be prepared. Culturing the RPE cells before harvesting is also preferred both to allow for the production of larger populations of RPE cells from a small amount of harvested tissue and to allow for a period of observation to ensure that the RPE cells to be transplanted are healthy and functional.

For proper culturing, harvested RPE cells contained are apposed to a substrate to which they will attach and grow, and which is capable of being maintained in culture conditions appropriate for efficient growth of RPE cells. Apposition of RPE cells may be accomplished by pipetting a solution containing a population of RPE cells onto the substrate. Intact sheets of harvested RPE cells in a physiological solution may also be physically released via a wide-bore pipette onto the substrate and manipulated with a fine camel-hair brush if necessary. Since RPE cell cultures grow best at around 37° C, appropriate substrates are solids at this temperature. Attachment substrates which are suitable for growth of RPE cell cultures include plastic culture ware or glass culture chamber slides coated with collagen laminin or fibronectin for cell attachment.

Culturing harvested RPE cells can be accomplished by any suitable method for obtaining a confluent monolayer of RPE cells. Appropriate culturing techniques are described in Pfeffer, B.A., Chapter 10, "Improved Methodology for Cell Culture of Human and Monkey Retinal Pigment Epithelium", Progress in Retinal Research, vol. 10 (1991); Ed. by Osborn, N. and Chader. J., or Mayerson, P.L., et al., "An Improved Method for Isolation and Culture of Rat Retinal Pigment Epithelial Cells", Investigative Ophthalmology & Visual Science, Nov., 1985, 26; 1599-1609.

Fig. 2 depicts a schematic of the preparation and transplantation of a preferred embodiment of the implant according to the invention. An implant laminate 1 is formed as depicted in Fig. 2a. Gelatin 3 is preferred for use to provide monolayer support during transplantation because it is flexible, non-toxic to neural tissues, and because it dissolves away after exposure to body temperature (37° C). However, since RPE grows too slowly at temperatures below 37° C, gelatin is not an appropriate substrate for use in culturing RPE cells. Thus, a monolayer of RPE cells 5 is first cultured on a suitable substrate for growth. RPE cells will grow on plastic culture ware A. However, to aid in removal of the monolayer of RPE cells 5 from the culture ware A, the culture ware A may be coated with a substrate 9 for the RPE cells. The substrate 9 is in turn coated with a thin (@ 2-4 microns) layer of collagen 7, to which the monolayer of RPE cells 5 readily attach, and which will serve as an anchor for the RPE cells in the post-transplantation period.

After a satisfactory culture of RPE cells has been grown, a 5 to 30% solution of molten gelatin is applied to the apical surface of the monolayer of RPE cells 5 in the culture. The culture ware containing the RPE cells and gelatin solution is then cooled so that the gelatin 3 is solidified into a sheet of preferably about 150-250 mm in thickness, attached to the apical surface of the RPE cells 5. The implant laminate 1 comprising a monolayer of RPE cells 5, attached to the collagen anchor 7 and an over layer of gelatin 3, may then be physically removed from the culture ware A by slicing between physically removed from the culture ware A by slicing between the culture ware A surface and the collagen 7 with a razor C, as depicted in Fig. 2a.

As depicted in Fig. 2b, the implant laminate 1 is cut to create an implant 1a sized so that it will fit into a surgical instrument 20 which contains a carrier channel for protection of the implant 1a during the transplantation procedure. The implant 1a is transplanted to the subretinal area at the posterior pole 76 of the host eye after detachment of the retina, as portrayed in Fig. 2c, using surgical techniques more fully disclosed below. The gelatin over layer dissolves within hours after insertion of the implant 1a into the host eye. After transplantation, the retina reattaches, and the monolayer of RPE cells, anchored to the layer of collagen, is sandwiched between the choroid and the retina. See Fig. 2d.

To transplant the implant, the host eye is prepared so as to reduce bleeding and surgical trauma. A transcleral/transchoroidal surgical approach to the subretinal space is an example of a suitable approach and it will be understood that other surgical approaches, such as a transcorneal approach, may also be used. The preferred surgical approach in the human, Fig. 3, includes making a transverse incision 70 in a sclera and choroid 78 of sufficient size so as to allow insertion of a surgical instrument 20. The instrument 20 is advanced through the sclera and choroid 78 and to the ora serrata 74 as illustrated in Fig. 3. The instrument detaches the retina as it is advanced under the retina and into the sub-retinal space to the posterior pole 76 of the eye.

Preferably, an instrument comprising an elongate tube having a flat, wide cross-section may be used so that the implant may be drawn into the elongate tube for protection as it is transported through an appropriate sized incision in the sclera or choroid. The instrument is advanced to the ora serrata 74 of the host eye and if the instrument includes a lumen, the retina is detached by the gentle force of a perfusate such as a saline-like fluid, carboxymethylcellulose, or 1-2% hyluronic acid ejected from the lumen. Advantageously, the fluid may additionally contain anti-oxidants, anti-inflammation agents, anesthetics or agents that slow the metabolic demand of the host retina.

If the instrument does not include a lumen, the retina is detached by subretinal irrigation or by the walls

Once transplantation is completed and the monolayer of RPE cells has been stabilized in its proper position, an intraocular injection of an enzyme such as agarase (1 mg/5 ml vol. of vitreal chamber) if an agarose substrate is used may be administered to break down the substrate. Alternatively, the enzyme may be incorporated into a slow-release agent 11 such as ethylene-vinyl acetate copolymer (Elvax 40W, DuPont Chemical Co., Del.), which is added to the substrate 9 prior to transplantation. Methods for using biodegradable polymers as slow-release matrices for enzymes are known in the art, such as disclosed by Powell, E.M., Brain Research (1990) 515:309-311; Wise, et al.; Chapter 12, "Lactic/Glycolic Acid Polymers," Drug Carriers in Biology and Medicine (Ed., Gregoriades), 1979; Kitchell, J. and Wise, D., "Poly(lactic/glycolic acid) Biodegradable Drug - Polymer Matrix Systems," Methods in Enzymology, 112:436-448 (1985). Slow release of the enzyme causes gradual break up of the substrate support. The collagen 7 remaining after dissipation of the substrate 9 is physiologically permeable to and does not impede normal tissue function, and serves to anchor the monolayer of RPE cells 5, as well as to inhibit neovascularization in the subretinal area of the host eye.

Fibrin, discussed in connection with Fig. 2, may also be used as the substrate 9 in the embodiment depicted in Fig. 3. Fibrin is the fibrous, insoluble protein that forms the structural component for blood clots. Fibrin may be pressed to add tensile strength and is a material which provides a flexible, non-toxic support to which RPE cells will attach and grow as a monolayer. To prepare fibrin, fibrin clot suitable for substrate use is made by catalyzing the polymerization of fibrinogen into fibrin by the addition of thrombin. After transplantation, fibrin will dissipate upon exposure to naturally produced enzymes which dissolve blood clots. If the dissipation of the fibrin support composition is to be accelerated, enzymes which are specific for fibrin, such as tissue plasminogen activator (TPA), urokinase or streptokinase may be administered using one of the methods for application of enzymes discussed above.

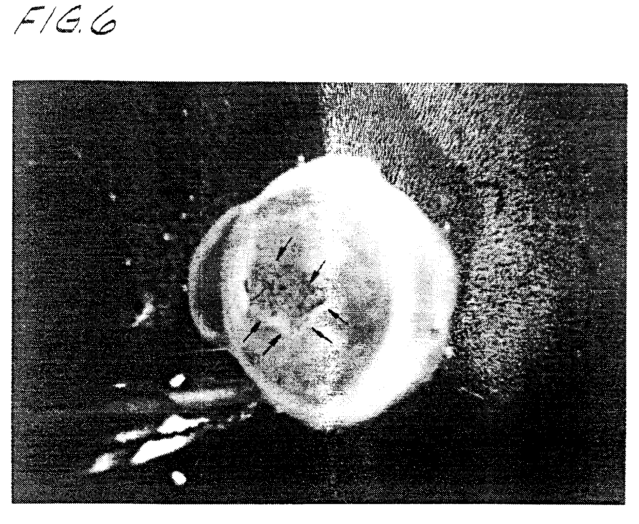

To transplant the implant, the host eye is prepared so as to reduce bleeding and surgical trauma. A transcleral/transchoroidal surgical approach to the subretinal space is an example of a suitable approach and it will be understood that other surgical approaches, such as a transcorneal approach, may also be used. The preferred surgical approach in the human, Fig. 4, includes making a transverse incision 70 in a sclera and choroid 78 of sufficient size so as to allow insertion of a surgical instrument 20. The instrument 20 is advanced through the sclera and choroid 78 and to the ora serrata 74 as illustrated in Fig. 4. The instrument detaches the retina as it is advanced under the retina and into the sub-retinal space to the posterior pole 76 of the eye.

Preferably, an instrument comprising an elongate tube having a flat, wide cross-section may be used so that the implant may be drawn into the elongate tube for protection as it is transported through an appropriate sized incision in the sclera or choroid. The instrument is advanced to the ora serrata 74 of the host eye and if the instrument includes a lumen, the retina is detached by the gentle force of a perfusate such as a saline-like fluid, carboxymethylcellulose, or 1-2% hyluronic acid ejected from the lumen. Advantageously, the fluid may additionally contain anti-oxidants, anti-inflammation agents, anesthetics or agents that slow the metabolic demand of the host retina.

If the instrument does not include a lumen, the retina is detached by subretinal irrigation or by the walls of the surgical instrument as it is advanced under the retina and into the subretinal space to the posterior pole 76 of the eye. The implant is then transplanted by retracting the tube containing the implant from the eye while simultaneously gently ejecting the implant from the tube. The instrument is then carefully withdrawn out of the eye. Retinal reattachment occurs rapidly and the monolayer of RPE cells is held in place in a sandwich-like arrangement between the retina and the choroid. The incision may require suturing.

Fig. 5 depicts a transcorneal surgical approach as an alternative to the transscleral and choroidal approaches described above. Except for the point of entry, the surgical technique is essentially the same as outlined for the transscleral or choroidal approaches. A transverse incision 70 is made in a cornea 72 and the instrument 20 containing the implant is advanced under the iris, through the cornea 72 and to the ora serrata 74 as illustrated in Fig. 5. The iris should be dilated for example, with topical atropine. The edges of the corneal incision are abutted and sutured if necessary to allow healing. The transcorneal approach is preferred for rodents because it has been found to reduce bleeding and surgical trauma. Nevertheless, a transscleral or choroidal approach is preferred for humans to avoid scarring of the cornea which may interfere with visual acuity.

A further surgical approach is to diathermize in the pars planna region to eliminate bleeding. The sclera is then incised and the choroidal and any native epithelial tissue is diathermized. The surgical tool is then inserted through the incision, the retina is intercepted at the ora serrata and the implant is deposited in the subretinal area otherwise as outlined elsewhere herein.

In yet a further surgical approach, entry is gained through the pars planna area as outlined above and an incision is made in the retina adjacent to the retinal macula. The surgical tool is then inserted through the retinotomy and into the macular area.

As discussed previously in connection with the make up of the support compositions used in accordance with the invention, after transplantation, the implant is exposed to a predetermined set of conditions, such as exposure to heat, selected enzymes or bodily fluids. Upon the appropriate amount of exposure, the support composition is dissipated or will not otherwise impede normal eye tissue function of the host eye and the transplanted population of RPE cells.

The following examples illustrates the invention.

### EXAMPLE 1

### Experimental Animal and Materials

RPE cells were taken from the sub-retinal area of donated human eyes (obtained from the Missouri Lions and St. Louis Eye Banks) following corneal removal. Hosts were adult albino rats immune-suppressed with cyclosporin A 10 mg/kg/day IP injection).

### Harvesting of RPE Cells

Immediately after corneal removal, the eye was pinned by the optic nerve stump into an eye cage and placed into an eye jar. The jar was flooded to capacity with cold Delbecco's modified essential medium (DMEM), supplemented with 5% fetal bovine serum. The eye was processed in a sterile environment. After loose connective tissue and muscle were carefully trimmed from the eye, it was placed upright on a sterile plate and the anterior segment with the adherent vitreous was lifted out of the eye cup. Four slits were cut radially toward the optic disc with the eye lying flat in a petri dish. The neural retina was then separated at the optic disc and removed. The shell was then washed with Hanks' balanced salt solution, Ca++ and Mg++ free, and treated with 0.25% trypsin for 30 min. at 37° C. The trypsin solution was aspirated from the shell and DMEM (GIBCO) was added. The cells were released from Bruch's membrane by gently brushing the surface of the RPE cells with the polished tip of a pasteur pipette and pipetting the culture medium in the shell.

### Culturing of RPE Cells

Glass culture chamber slides were prepared by coating them with liquid collagen obtained from Sigma Chemical Co., St. Louis. The collagen was allowed to solidify to about 150 microns in thickness. 3X10⁵ RPE cells were plated onto the prepared slides using a pasteur pipette. The RPE cells were then incubated in DMEM + F12 (1:1. GIBCO) supplemented with 20% fetal bovine serum (Sigma Chemical Co., St. Louis). The culture medium was changed every 3-4 days until a confluent monolayer of RPE cells had been cultivated. Implants comprising monolayers of RPE cells laminated to collagen were removed from the culture chamber slides and sheets of approximately 2 mm X 4 mm were cut out for transplantation.

### Surgical Procedure

A transverse incision was made in the cornea sufficient to allow insertion of a surgical instrument that is 2.5 mm wide with a lumen 0.5 mm high. The instrument was advanced under the iris (dilated with topical atropine) to the ora serrata, detaching the retina. The carrier was then advanced under the retina into the subretinal space to the posterior pole of the eye. The instrument allowed an implant comprising a monolayer of RPE cells apposed to a 150 mm collagen substrate (up to 2.5 X 4 mm) to be guided into the retinal space by advancement of the plunger with simultaneous retraction of the surgical instrument. The instrument was then removed. Following removal of the instrument, the edges of the corneal incision were abutted to allow rapid, sutureless healing. The eye was patched during recovery and a prophylactic dose of penicillin was administered. Upon removal of the patch, a veterinary ophthalmic antibiotic ointment was applied.

Transplant recipients were maintained on a 12 hr/12 hr light/dark cycle with an average light intensity of 50 lux. Following appropriate survival times, the animal was overdosed with pentobarbital.

Fig. 6 shows a view of the transplanted monolayer of RPE cells positioned under the retina, with the cornea, pupil and lens removed, two weeks post-transplantation. The arrows designate the edges of the implant.

### Histologic Preparation

For histologic evaluation, rat eyes with RPE cell implants were immersion-fixed in Bouin's solution for 5 hrs., then dehydrated in a graded ethanol series. The eyes were trimmed with a razor blade to include the transplanted area for implanted retinas, then processed through xylene and embedded in paraffin. Sections were cut at 8mm and stained with hematoxylin and eosin.

### Results

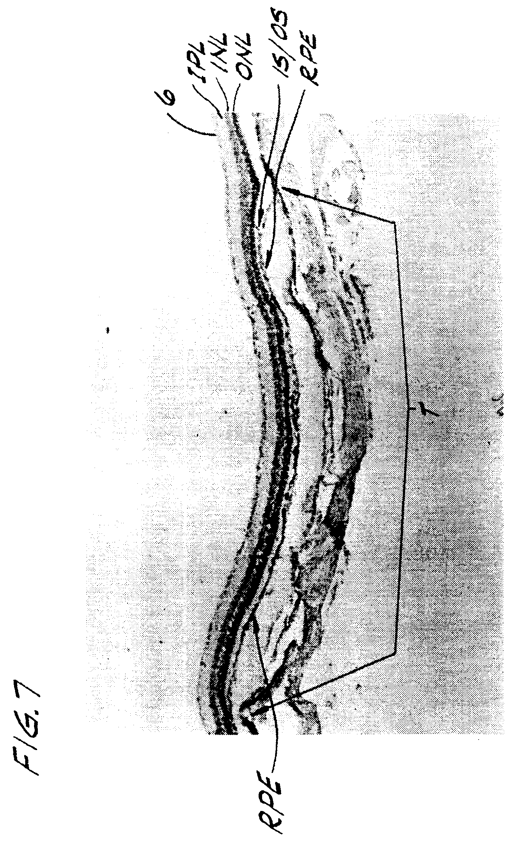

By using the transcorneal approach, it was found that the positioning of the monolayer of RPE cells between the host's retina and the adjacent choroidal tissue layer of the eye could be accomplished while minimizing the vascular damage and subsequent bleeding into the eye. In addition, it was found that this approach does not appear to disrupt the integrity of the retina, which reattaches to the back of the eye with the transplanted RPE cells interposed between the retina and the choroid. Using this insertion method, it was possible to position the RPE cells beneath the posterior pole of the retina (Fig. 7).

To determine the viability of the transplanted RPE cells, paraffin sections (8 mm) were made from the eye receiving the RPE cell transplant at 2 weeks, 4 weeks, and 3 months after transplantation. It was found that the monolayer of RPE cells survived transplantation at all times tested (10 out of 12 transplants).

With immune-suppression, successful transplants were seen at all survival times so far examined (2 weeks to 3 months), showing apparent physical integration with the host eye and maintaining morphological features of the RPE as illustrated in Fig. 7 which shows a transplant of a monolayer of human RPE cells from adult donor to adult rat host. (T, transplant). 40X.

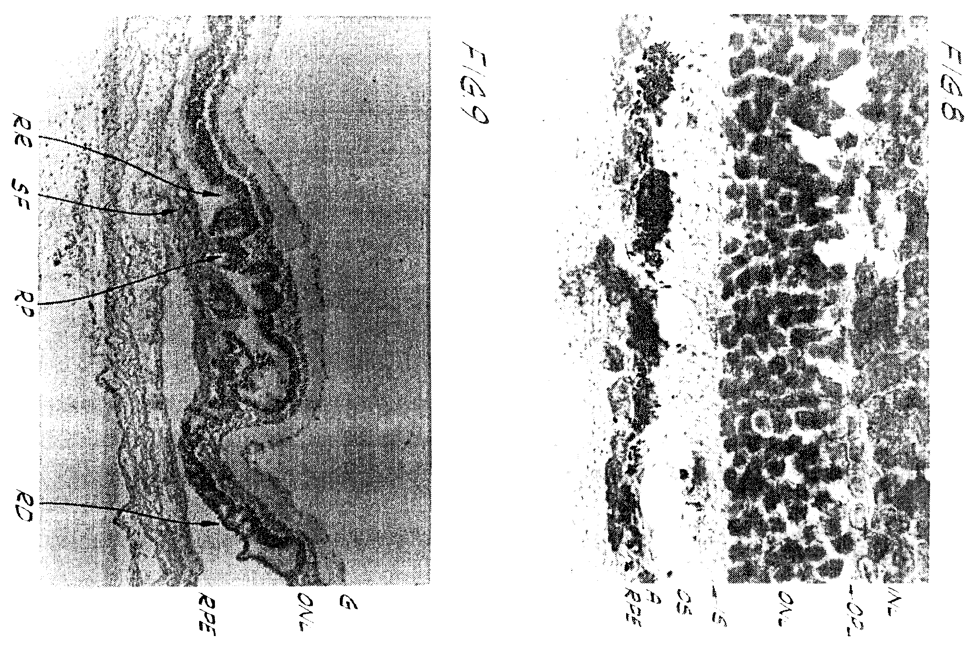

Paraffin sections made at 2 weeks post-transplantation are shown in Figs. 7 and 8. Fig. 7 is a low-power photomicrograph showing the location of the RPE monolayer transplant (T) between arrowheads at the posterior pole of th host eye. Note the maintenance of normal retinal configuration as well as the normal appearance of the RPE cells, maintained in substantially a monolayer between the choroid and the outer segments of the photoreceptor cells. Figure 8 is a higher-power photomicrograph showing in detail the interface between the transplant and the adjacent retina. As shown in Fig. 8, the photoreceptor outer segments are shown to be in normal apposition to the apical microvillar processes (A) of transplanted RPE cells.

The functional capabilities of the transplanted RPE cells and reconstructed retina were ascertained by their maintenance of photoreceptor bodies, and the presence of inner and outer segments with proper orientation to the apical processes of transplanted RPE cells. The success of these procedures for transplanting monolayers of RPE cells are reflected in Figs. 7 and 8.

### EXAMPLE 2

As a comparison, mature human RPE cells were transplanted in dissociated form using the bolus injection method as set forth by Li and Turner in Exp. Eye Res., 47:911 (1988). RPE cells from the same donor tissue used for the transplants conducted as set forth in Example 1 were harvested and cultured as set forth in Example 1. After culturing, the RPE cells were trypsonized to form dissociated RPE cells for transplantation by bolus injection according to the method of Li and Turner. Hosts were adult albino rats immune-suppressed with cyclosporin A 10 mg/kg/day IP injection) as set forth in Example 1.

Transplant recipients were maintained on a 12 hr/12 hr light/dark cycle with an average light intensity of 50 lux. Following appropriate survival times, the animal was overdosed with pentobarbital. Paraffin sections of the eye receiving the RPE cell implant were then cut (8 mm).

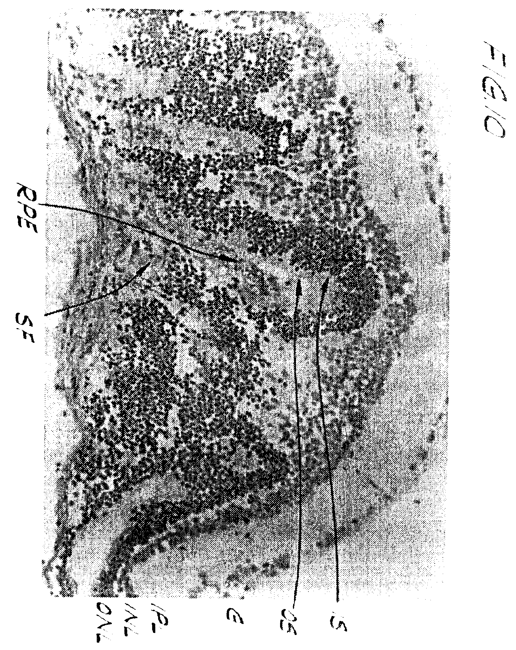

Paraffin sections made at 2 weeks post-transplantation are shown in Figs. 9 and 10. Fig. 9 is a low-power photomicrograph showing the location of the injection of dissociated RPE cells at the posterior pole of the host eye. Note the pathological configurations, including retinal detachment (RD), retinal pucker (RP), subretinal fibrosis (SF) and retinal rosette (RR) formation. Figure 10 is a higher-power photomicrograph showing in detail the pathological invasion of RPE cells into the adjacent retina, subretinal fibrosis and retinal pucker.

From the foregoing description those skilled in the art will appreciate that all aspects of the present invention are realized. The present invention provides an improved surgical implant that is adapted to provide cell organization during transplantation of the RPE cells. With the implant of this invention cell organization is maintained during and after RPE transplantation.

In view of the above, it will be seen that the several objects of the invention are achieved and other advantages attained.

As various changes could be made in the above compositions and methods without departing from the scope of the invention, it is intended that all matter contained in the above description or shown in the accompanying drawings shall be interpreted as illustrative and not in a limiting sense.

## Claims

1. A method for the preparation of a population of Retinal Pigment Epithelium cells for transplantation to the subretinal area of an eye of a host comprising :
a) providing donor tissue comprising Retinal Pigment Epithelium cells,
b) harvesting Retinal Pigment Epithelium cells from the donor tissue,
c) culturing the harvested Retinal Pigment Epithelium cells upon a culture surface having thereon a substrate suitable for attachment and growth of a monolayer of Retinal Pigment Epithelium cells, wherein said substrate can be removed along with said monolayer.
d) removing the monolayer of cultured Retinal Pigment Epithelium cells with the substrate from the culture surface.

2. A method for the preparation of a population of Retinal Pigment Epithelium cells for transplantation to the subretinal area of an eye of a host, according to claim 1, wherein the monolayer of Retinal Pigment Epithelium cells are apposed to a non-toxic flexible support that, upon transplantation to the subretinal area and exposure to conditions which result in a breakdown of at least a portion of the non-toxic flexible support, will not impede normal eye tissue function of the eye of the host and the transplanted population.

3. A method as set forth in claim 1 wherein the donor tissue comprises mature Retinal Pigment Epithelium cells.

4. A method as set forth in claim 1 wherein the donor tissue is autologous to the host.

5. A method as set forth in claim 1 wherein said substrate comprises a layer of collagen less than about 100 microns in thickness.

6. A method as set forth in claim 1 wherein the Retinal Pigment Epithelium cells rave apical and basal surfaces, the substrate comprises collagen and the support comprises an over layer of gelatin, and the basal surfaces of the harvested Retinal Pigment Epithelium cells are apposed to the collagen and the apical surfaces of the harvested Retinal Pigment Epithelium cells are apposed to the gelatin.

7. A method as set forth in claim 1 wherein the harvested Retinal Pigment Epithelium cells have an apical surface and apposition of the Retinal Pigment Epithelium cells to the support further comprises contacting the apical surface of the Retinal Pigment Epithelium cells to the support.

8. A method as set forth in claim 1 wherein said support comprises a material degradable by exposure to heat, or bodily fluids.

9. A method as set forth in claim 1 wherein one or both of the support and the substrate comprises one or more of a trophic factor, an immunosuppressant, an anti-inflammation agent, an anti-angiogenic factor, an anti-glial agent or an anti-mitotic factor.

10. A method for the preparation of a population of Retinal Pigment Epithelium cells for transplantation to the subretinal area of an eye of a host comprising :
a) providing donor tissue comprising Retinal Pigment Epithelium cells,
b) harvesting Retinal Pigment Epithelium cells from the donor tissue,
c) culturing the harvested Retinal Pigment Epithelium cells upon a culture surface having thereon a substrate suitable for attachment and growth of a monolayer of Retinal Pigment Epithelium cells, wherein said substrate can be removed along with said monolayer
d) removing the monolayer of cultured Retinal Pigment Epithelium cells with the substrate from the culture surface,
e) apposing the monolayer of cultured Retinal Pigment Epithelium cells to a non-toxic flexible support that, upon transplantation to the subretinal area and exposure to conditions which result in a breakdown of at least a portion of the non-toxic, flexible support, will not impede normal eye tissue function of the eye of the host and the transplanted population.

11. An implant for transplantation to the subretinal area of an eye of a host of a monolayer of Retinal Pigment Epithelium cells, characterized in that said Retinal Pigment Epithelium cells comprise cultured Retinal Pigment Epithelium cells.

12. An implant as set forth in claim 11, wherein said cultured Retinal Pigment Epithelium cells are laminated on a thin collagen substrate.

13. An implant for transplantation to the subretinal area of an eye of a host, according to claim 11, characterised in that it comprises a laminate of a monolayer of Retinal Pigment Epithelium cells and a non-toxic, flexible support that, upon transplantation to the subretinal area and exposure to conditions which result in a breakdown of at least a portion of the non-toxic, flexible support, will not impede normal eye tissue function.

14. An implant as set forth in claim 11, wherein the Retinal Pigment Epithelium cells further comprise mature Retinal Pigment Epithelium cells.

15. An implant as set forth in claim 11, wherein the Retinal Pigment Epithelium cells are autologous to the host.

16. An implant as set forth in claim 11, wherein the support comprises a material degradable by exposure to heat or bodily fluids.

17. An implant as set forth in claim 11, wherein the support comprises a layer of collagen less than about 100 microns in thickness.

18. An implant as set forth in claim 11, wherein the monolayer of Retinal Pigment Epithelium cells have apical and basal surfaces, the support further comprises a substrate of collagen and an over layer of gelatin, and the basal surfaces of the monolayer of Retinal Pigment Epithelium cells are laminated to the collagen and the apical surfaces of the monolayer of Retinal Pigment Epithelium cells are laminated to the gelatin.

19. An implant as set forth in claim 11 wherein the support comprises one or more of a trophic factor, an immunosuppressant, an anti-inflammation agent, an anti-angiogenic factor, an anti-glial agent or an anti-mitotic factor.

## Patentansprüche

1. Verfahren zur Herstellung einer Population von Retinapigmentepithel-Zellen zur Transplantation in den subretinalen Bereich eines Empfängerauges umfassend die Stufen:
(a) Bereitstellen von Spendergewebe, das Retinapigmentepithel-Zellen umfaßt,
(b) Gewinnen der Retinapigmentepithel-Zellen aus dem Spendergewebe,
(c) Kultivieren der gewonnenen Retinapigmentepithel-Zellen auf einer Kulturoberfläche, die darauf ein Substrat aufweist, das zur Anhaftung und zum Wachstum einer Monoschicht von Retinapigmentepithel-Zellen geeignet ist, wobei das Substrat zusammen mit der Monoschicht entfernt werden kann,
(d) Entfernen der Monoschicht der kultivierten Retinapigmentepithel-Zellen zusammen mit dem Substrat von der Kulturoberfläche.

2. Verfahren zur Herstellung einer Population von Retinapigmentepithel-Zellen zur Transplantation in den subretinalen Bereich eines Empfängerauges nach Anspruch 1, dadurch gekennzeichnet, daß die Monoschicht der Retinapigmentepithel-Zellen an einem nicht-toxischen flexiblen Träger angebracht werden, der bei der Transplantation in den subretinalen Bereich und dem Ausgesetztsein gegenüber Bedingungen, die zu einem Abbau von mindestens einem Teil des nicht-toxischen flexiblen Trägers führen, die Funktion des normalen Augengewebes des Empfängerauges und die transplantierte Population nicht nachteilig beeinflußt.

3. Verfahren nach Anspruch 1, dadurch gekennzeichnet, daß das Spendergewebe reife Retinapigmentepithel-Zellen umfaßt.

4. Verfahren nach Anspruch 1, dadurch gekennzeichnet, daß das Spendergewebe gegenüber dem Empfängerauge autolog ist.

5. Verfahren nach Anspruch 1, dadurch gekennzeichnet, daß das Substrat eine Kollagenschicht mit einer Dicke von weniger als ca. 100 µm aufweist.

6. Verfahren nach Anspruch 1, dadurch gekennzeichnet, daß die Retinapigmentepithel-Zellen apicale und basale Oberflächen haben, das Substrat Kollagen aufweist, und der Träger eine Deckschicht aus Gelatine aufweist, und die basalen Oberflächen der gewonnenen Retinapigmentepithel-Zellen am Kollagen angebracht sind, und die apicalen Oberflächen der gewonnenen Retinapigmentepithel-Zellen an der Gelatine angebracht sind.

7. Verfahren nach Anspruch 1, dadurch gekennzeichnet, daß die gewonnenen Retinapigmentepithel-Zellen eine apicale Oberfläche aufweisen, und das Anbringen der Retinapigmentepithel-Zellen an den Träger außerdem das In-Kontakt-bringen der apicalen Oberfläche der Retinapigmentepithel-Zellen mit dem Träger umfaßt.

8. Verfahren nach Anspruch 1, dadurch gekennzeichnet, daß der Träger ein Material umfaßt, das beim Einwirken von Hitze oder Körperflüssigkeiten abbaubar ist.

9. Verfahren nach Anspruch 1, dadurch gekennzeichnet, daß der Träger oder das Substrat oder beide eine oder mehrere Substanzen bestehend aus einem trophischen Faktor, einem Immunosuppressivum, einem entzündungshemmenden Mittel, einem antiangiogenen Faktor, einem antiglialen Mittel oder einem antimitotischen Faktor umfassen.

10. Verfahren zur Herstellung einer Population von Retinapigmentepithel-Zellen zur Transplantation in den subretinalen Bereich eines Empfängerauges, umfassend die Stufen:
(a) Bereitstellen eines Spendergewebes, das Retinapigmentepithel-Zellen umfaßt,
(b) Gewinnen der Retinapigmentepithel-Zellen aus dem Spendergewebe,
(c) Kultivieren der gewonnenen Retinapigmentepithel-Zellen auf einer Kulturoberfläche, die darauf ein Substrat aufweist, das zur Anhaftung und zum Wachstum einer Monoschicht von Retinapigmentepithel-Zellen geeignet ist, wobei das Substrat zusammen mit der Monoschicht entfernt werden kann,
(d) Entfernen der Monoschicht der kultivierten Retinapigmentepithel-Zellen zusammen mit dem Substrat von der Kulturoberfläche, und
(e) Anbringen der Monoschicht der kultivierten Retinapigmentepithel-Zellen an einem nicht-toxischen flexiblen Träger, der bei der Transplantation in den subretinalen Bereich und dem Ausgesetztsein gegenüber Bedingungen, die zu einem Abbau von mindestens einem. Teil des nicht-toxischen flexiblen Trägers führen, die Funktion des normalen Augengewebes des Empfängerauges und die transplantierte Population nicht nachteilig beeinflußt.

11. Implantat zur Transplantation in den subretinalen Bereich eines Empfängerauges aus einer Monoschicht von Retinapigmentepithel-Zellen, dadurch gekennzeichnet, daß die Retinapigmentepithel-Zellen kultivierte Retinapigmentepithel-Zellen umfassen.

12. Implantat nach Anspruch 11, dadurch gekennzeichnet, daß die kultivierten Retinapigmentepithel-Zellen auf einem dünnen Kollagensubstrat auflaminiert sind.

13. Implantat zur Transplantation in den subretinalen Bereich eines Empfängerauges nach Anspruch 11, dadurch gekennzeichnet, daß es ein Laminat einer Monoschicht von Retinapigmentepithel-Zellen und eines nicht-toxischen flexiblen Trägers umfaßt, der bei der Transplantation in dem subretinalen Bereich und dem Ausgesetztsein gegenüber Bedingungen, die zu einem Abbau von mindestens einem Teil des nicht-toxischen flexiblen Trägers führen, die Funktion des normalen Augengewebes nicht nachteilig beeinflußt.

14. Implantat nach Anspruch 11, dadurch gekennzeichnet, daß die Retinapigmentepithel-Zellen außerdem reife Retinapigmentepithel-Zellen umfassen.

15. Implantat nach Anspruch 11, dadurch gekennzeichnet, daß die Retinapigmentepithel-Zellen gegenüber dem Empfänger autolog sind.

16. Implantat nach Anspruch 11, dadurch gekennzeichnet, daß der Träger ein Material umfaßt, das bei Einwirkung von Hitze oder Körperflüssigkeiten abbaubar ist.

17. Implantat nach Anspruch 11, dadurch gekennzeichnet, daß der Träger eine Schicht aus Kollagen mit einer Dicke von weniger als ca. 100 µm aufweist.

18. Implantat nach Anspruch 11, dadurch gekennzeichnet, daß die Monoschicht der Retinapigmentepithel-Zellen apicale und basale Oberflächen aufweist, der Träger außerdem ein Substrat aus Kollagen und eine Deckschicht aus Gelatine, aufweist und die basalen Oberflächen der Monoschicht der Retinapigmentepithel-Zellen auf dem Kollagen auflaminiert sind und die apicalen Oberflächen der Monoschicht der Retinapigmentepithel-Zellen auf der Gelatine auflaminiert sind.

19. Implantat nach Anspruch 11, dadurch gekennzeichnet, daß der Träger eine oder mehrere Substanzen bestehend aus einem trophischen Faktor, einem Immunosuppressivum, einem entzündungshemmenden Mittel, einem antiangiogenen Faktor, einem antiglialen Mittel oder einem antimitotischen Faktor umfaßt.

## Revendications

1. Une méthode pour la préparation d'une population de cellules d'épithélium pigmentaire de la rétine en vue de sa transplantation dans la région subrétinienne de l'oeil d'un receveur, cette méthode consistant à :
a) obtenir d'un donneur du tissu contenant des cellules d'épithélium pigmentaire de la rétine ;
b) recueillir les cellules d'épithélium pigmentaire de la rétine de ce tissu du donneur ;
c) cultiver les cellules d'épithélium pigmentaire de la rétine recueillies sur une surface de culture portant un substrat approprié à la fixation et à la croissance d'une monocouche de cellules d'épithélium pigmentaire de la rétine, ledit substrat pouvant être enlevé en même temps que ladite monocouche ;
d) prélever de la surface de culture la monocouche de cellules d'épithélium pigmentaire de la rétine cultivées avec le substrat.

2. Une méthode pour la préparation d'une population de cellules d'épithélium pigmentaire de la rétine en vue de sa transplantation dans la région subrétinienne de l'oeil d'un receveur selon la revendication 1, dans laquelle la monocouche de cellules d'épithélium pigmentaire de la rétine est apposée sur un support souple non toxique qui, lors de la transplantation dans la région subrétinienne et d'une exposition à des conditions entraînant une rupture d'au moins une portion du support souple non toxique, n'entrave pas le fonctionnement normal du tissu de l'oeil chez l'oeil du receveur et de la population transplantée.

3. Une méthode selon la revendication 1, dans laquelle le tissu du donneur est constitué par des cellules d'épithélium pigmentaire de la rétine arrivées à maturité.

4. Une méthode selon la revendication 1, dans laquelle le tissu du donneur est autologue vis à vis du receveur.

5. Une méthode selon la revendication 1, dans laquelle ledit substrat est constitué par une couche de collagène d'une épaisseur inférieure à environ 100 microns.

6. Une méthode selon la revendication 1, dans laquelle les cellules d'épithélium pigmentaire de la rétine ont des surfaces apicales et basales, le substrat est constitué par du collagène et le support est constitué par une sur-couche de gélatine, les surfaces basales des cellules d'épithélium pigmentaire de la rétine recueillies sont apposées sur le collagène, et les surfaces apicales des cellules d'épithélium pigmentaire de la rétine recueillies sont apposées sur la gélatine.

7. Une méthode selon la revendication 1, dans laquelle les cellules d'épithélium pigmentaire de la rétine ont une surface apicale et l'apposition des cellules d'épithélium pigmentaire de la rétine au support consiste au surplus à mettre en contact la surface apicale des cellules d'épithélium pigmentaire de la rétine avec le support.

8. Une méthode selon la revendication 1, dans laquelle ledit support est constitué par une matière dégradable par exposition à la chaleur ou aux fluides corporels.

9. Une méthode selon la revendication 1, dans laquelle le support, soit le substrat, soit les deux, sont constitués par un ou plusieurs facteurs trophiques, un agent immunosuppresseur, un agent anti-inflammatoire, un facteur antiangiogène, un agent antiglial ou un facteur antimitotique.

10. Une méthode pour la préparation d'une population de cellules d'épithélium pigmentaire de la rétine en vue de sa transplantation dans la région subrétinienne de l'oeil d'un receveur, celle méthode consistant à :
a) obtenir d'un donneur du tissu contenant des cellules d'épithélium pigmentaire de la rétine ;
b) recueillir les cellules d'épithélium pigmentaire de la rétine de ce tissu du donneur ;
c) cultiver les cellules d'épithélium pigmentaire de la rétine recueillies sur une surface de culture portant un substrat approprié à la fixation et à la croissance d'une monocouche de cellules d'épithélium pigmentaire de la rétine, ledit substrat pouvant être enlevé en même temps que ladite monocouche ;
d) prélever sur la surface de culture la monocouche de cellules d'épithélium pigmentaire de la rétine cultivées avec le substrat ; et
e) apposer la monocouche de cellules d'épithélium pigmentaire de la rétine cultivées sur un support souple non toxique qui, par transplantation dans la région subrétinienne et exposition à des conditions entraînant une rupture d'au moins une portion du support souple non toxique, n'entrave pas le fonctionnement normal du tissu de l'oeil du receveur et de la population transplantée.

11. Un implant destiné à une transplantation, dans la région subrétinienne de l'oeil d'un receveur, d'une monocouche de cellules d'épithélium pigmentaire de la rétine, caractérisé en ce que lesdites cellules d'épithélium pigmentaire de la rétine consistent en cellules cultivées d'épithélium pigmentaire de la rétine.

12. Un implant selon la revendication 11, dans lequel lesdites cellules d'épithélium pigmentaire de la rétine cultivées sont stratifiées sur un substrat mince en collagène.

13. Un implant destiné à une transplantation dans la région subrétinienne d'un receveur selon la revendication 11, caractérisé en ce qu'il consiste en une stratification d'une monocouche de cellules d'épithélium pigmentaire de la rétine avec un support souple non toxique qui, par transplantation dans la région subrétinienne et exposition à des conditions entraînant une rupture d'au moins une portion du support souple non toxique, n'entrave pas le fonctionnement normal du tissu de l'oeil.

14. Un implant selon la revendication 11, dans lequel les cellules d'épithélium pigmentaire de la rétine consistent en cellules d'épithélium pigmentaire de la rétine arrivées à maturité.

15. Un implant selon la revendication 11, dans lequel les cellules d'épithélium pigmentaire de la rétine sont autologues à l'égard du receveur.

16. Un implant selon la revendication 11, dans lequel le support est constitué par une matière dégradable par exposition à la chaleur ou aux fluides corporels.

17. Un implant selon la revendication 11, dans lequel le support est constitué par une couche de collagène d'une épaisseur inférieure à environ 100 microns.

18. Un implant selon la revendication 11, dans lequel la monocouche de cellules d'épithélium pigmentaire de la rétine ont des surfaces apicales et basales, le support se compose au surplus d'un substrat en collagène et d'une sur-couche de gélatine, les surfaces basales de la monocouche de cellules d'épithélium pigmentaire de la rétine sont stratifiées avec le collagène et les surfaces apicales de la monocouche de cellules d'épithélium pigmentaire de la rétine sont stratifiées avec la gélatine.

19. Un implant selon la revendication 11, dans lequel le support contient un ou plusieurs des facteurs suivants : un facteur trophique, un agent immunosuppresseur, un agent anti-inflammatoire, un facteur antigène, un agent antiglial et un facteur antimitotique.
